# DEMANDE DE BREVET EUROPEEN

(11) **EP 1 400 237 A2**
(43) Date de publication de la demande: **24.03.2004**
(21) Numéro de dépôt: 03292186.8
(22) Date de dépôt: 05.09.2003
(51) Int. Cl.: A61K 7/48, A61K 7/06

(54) **Utilisation d'un activateur de protectin pour améliorer la résistance de la peau**

(30) Priorité: 06.09.2002 FR 0211078
(71) Demandeur: L'OREAL, 75008 Paris (FR)
(72) Inventeur: Mahe, Yann, 91390 Morsang sur Orge (FR); Meybeck, Alain, 92400 Courbevoie (FR)
(74) Mandataire: Allab, Myriam

(57) **Abrégé**

L'invention concerne l'utilisation d'au moins un activateur de la production de Protectin ou de précurseur de Protectin dans une composition cosmétique ou pour la préparation d'une composition destinée à limiter les effets secondaires de l'activation du complément sur la peau, les muqueuses et/ou le cuir chevelu. Elle concerne également des compositions contenant de tels activateurs, un procédé de sélection d'un produit capable de protéger les membranes cellulaires et un procédé cosmétique pour améliorer la résistance de la peau aux effets de l'activation du complément.

## Description

La présente invention concerne l'utilisation de substances renforçant la résistance de la peau en activant la production de Protectin ou précurseur de Protectin par les cellules cutanées, notamment dans des compositions cosmétiques ou dermatologiques. Elle se rapporte également à des compositions contenant de telles substances et à un procédé de sélection de composés utilisables dans ces compositions.

La peau constitue une barrière contre les agressions extérieures, notamment chimiques, mécaniques ou infectieuses, et à ce titre un certain nombre de réactions de défense contre les facteurs environnementaux (climat, rayons ultraviolets, tabac, ...) et/ou les xénobiotiques, comme par exemple les micro-organismes, se produisent à son niveau. Ces mécanismes de défense sont chez l'individu en bonne santé efficaces pour éviter la propagation d'infections.

Cependant, ces mécanismes de défense, s'ils sont bénéfiques pour l'organisme, peuvent entraîner des réactions indésirables localement, qui peuvent consister en un simple inconfort ou modification de l'apparence, jugés inesthétiques par le sujet qui le ressent. Dans d'autres cas, ces réactions locales peuvent être excessives et entraîner elles-mêmes des dérèglements du fonctionnement physiologique.

Il serait donc souhaitable de pouvoir disposer d'actifs qui protégeraient la peau des effets indésirables des réactions de défense de l'organisme, sans toutefois entraver le fonctionnement de ces mécanismes indispensables à sa protection contre les agents externes.

La peau est constituée de deux compartiments, l'un superficiel, l'épiderme, et un plus profond, le derme, qui interagissent. L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau, notamment le rôle de protection de l'organisme des agressions extérieures appelé "fonction barrière". L'épiderme est conventionnellement divisé en une couche basale de kératinocytes constituant la couche germinative de l'épiderme, une couche dite épineuse constituée de plusieurs couches de cellules polyédriques disposées sur les couches germinatives, une à trois couches dite granuleuses constituée de cellules aplaties contenant des inclusions cytoplasmiques distinctes, les grains de kératohyaline et enfin la couche cornée (ou *stratum corneum*), constituée d'un ensemble de couches de kératinocytes au stade terminal de leur différenciation appelés cornéocytes.
Le derme fournit à l'épiderme un support solide. C'est également son élément nourricier. Il est principalement constitué de fibroblastes et d'une matrice extracellulaire composée majoritairement de collagène, d'élastine et d'une substance, dite substance fondamentale. Ces composants sont synthétisés par les fibroblastes. On y trouve aussi des leucocytes, des mastocytes ou encore des macrophages tissulaires. Enfin, le derme est traversé par des vaisseaux sanguins et des fibres nerveuses.
La cohésion entre l'épiderme et le derme est assurée par la jonction dermo-épidermique. C'est une région complexe d'une épaisseur de 100 nm environ qui comprend le pôle basal des kératinocytes basaux, la membrane épidermique et la zone sous-basale du derme superficiel.

La protection contre les facteurs externes implique notamment l'activation du complément, par une voie spécifique d'un anticorps ou par une voie alterne constituant un moyen de défense immédiat. Le complément est constitué d'une vingtaine de protéines circulantes dont l'activation en cascade est à l'origine de l'apparition d'activités biologiques variées, telles que la lyse cellulaire, bactérienne ou virale. Dans des situations physiologiques, il contribue notamment à la protection bactérienne et à la coagulation des plaies. II a pour vocation d'être activé rapidement et de façon localisée. L'activation du complément contribue cependant de façon significative à la pathogénèse d'états inflammatoires et à certains mécanismes de réactions autoimmunes.

Ces phénomènes ont notamment pour origine l'attaque des membranes cellulaires par le complexe formé par l'activation du complément, lorsque son activation se retourne contre les propres constituants cellulaires de l'organisme agressé.

De manière inattendue, la demanderesse a maintenant trouvé que certains composés étaient capables d'activer spécifiquement la production endogène d'une protéine permettant de protéger les cellules, en particulier les cellules cutanées, contre ces attaques, sans pour autant diminuer les défenses de l'organisme contre les agents externes.

C'est pourquoi la présente invention a pour objet l'utilisation d'au moins un modulateur, en particulier un activateur, de la production de Protectin ou d'un précurseur de Protectin dans une composition cosmétique ou pour la préparation d'une composition, le modulateur ou la composition étant destinés à améliorer la résistance de la peau, des muqueuses et/ou du cuir chevelu aux effets secondaires non spécifiques de l'activation du complément. Avantageusement, la composition est destinée à être appliquée topiquement sur la peau, les muqueuses et/ou le cuir chevelu.

La Protectin, aussi appelée CD59, est une glycoprotéine naturellement présente dans la peau qui fait partie des régulateurs physiologiques du complément. Sa structure a notamment été décrite par Sawada et al (DNA Cell Biol, 1990, 9(3): 213-20). Elle a en particulier pour fonction de réguler à la baisse la formation du complexe d'attaque des membranes cellulaires. (Venneker et al, Exp Clin lmmunogenet, 1992, 33-47)

Les compositions selon l'invention renforceront la résistance de la peau, des muqueuses, ou des follicules pileux aux effets secondaires de l'activation du complément et amélioreront donc sa tolérance. Les composés modulant, et notamment stimulant la production endogène de Protectin ou de précurseur de Protectin ou les compositions les contenant sont ainsi destinés à limiter les effets cutanés non spécifiques de l'activation du complément. En particulier, il s'agira de compositions ou d'actifs apaisants et/ou anti-irritants. lls diminuent ainsi l'apparition de rougeurs ou de petits boutons disgracieux.

Les compositions et/ou les activateurs de la production endogène de Protectin ou précurseur de Protectin selon l'invention sont ainsi utiles pour prévenir, diminuer ou traiter l'érythème, en particulier l'érythème solaire. En effet, le rôle du complément dans les réactions accompagnant une exposition trop prolongée de la peau aux rayonnements UV, en particulier UV B a été montré, et la protection des membranes des cellules contre les complexes résultant de son activation réduira ses effets liés à une hyper réactivité cutanée.

Selon un autre aspect de l'invention, l'activateur ou la composition le contenant sont destinés à traiter les peaux acnéiques ou à tendance acnéique. Plus particulièrement, l'activateur ou la composition seront destinés au soin des peaux pré-acnéiques et/ou séborrhéiques pour prévenir ou atténuer les rougeurs liées à l'irritation du follicule pileux.

Les activateurs de la production endogène de Protectin ou de précurseur de Protectin seront utiles selon l'invention pour diminuer les signes de la couperose et de manière générale les manifestations inesthétiques de fragilité capillaire telles que l'apparition de rougeurs localisées en cas de frottements répétés, liés par exemple à des mouchages réitérés en cas de rhume.

Selon encore un autre des aspects de l'invention, les compositions ou les activateurs de la production endogène de Protectin ou de précurseur de Protectin sont destinés au traitement des peaux sensibles.

Les sujets dits à peaux sensibles ou à cuir chevelu sensible constituent un ensemble désormais bien identifié. lls se caractérisent par un ensemble de symptômes qui sont en particulier des signes subjectifs tels que des sensations dysesthésiques. On entend par sensations dysesthésiques des sensations plus ou moins douloureuses ressenties dans une zone cutanée comme les picotements, fourmillements, démangeaisons ou prurits, échauffements, inconforts, tiraillements, etc. Les peaux sensibles peuvent être scindées en deux grandes formes cliniques, les peaux irritables et/ou réactives, et les peaux intolérantes. Les autres signes pouvant être associés aux sensations dysesthésiques sont les rougeurs ou érythème, des dartres, et, dans le sous-groupe des peaux intolérantes une hyperséborrhée. La caractéristique essentielle de la peau sensible est, un mécanisme de réponse à des facteurs extérieurs, qui peut concerner tout individu, même si les individus dits à peau sensible y réagissent plus vite que les autres. Ce mécanisme n'est cependant pas immunologique, il est aspécifique.
Les cuirs chevelus "sensibles" ont une sémiologie clinique plus univoque : les sensations de prurit et/ou de picotements et/ou d'échauffements sont essentiellement déclenchés par des facteurs locaux tels que frottements, savon, tensioactifs, eau dure à forte concentration de calcaire, shampooings ou lotions. Ces sensations sont aussi parfois déclenchées par des facteurs tels que l'environnement, les émotions et/ou les aliments. Un érythème et une hyperséborrhée du cuir chevelu ainsi qu'un état pelliculaire sont fréquemment associés aux signes précédents. Pour déterminer si une peau est sensible ou non, on peut effectuer un test à la capsaïcine, tel que décrit notamment dans le brevet EP 723 774.

Une peau sensible n'est donc pas une peau allergique. En effet, une peau allergique est une peau qui réagit à un agent extérieur, un allergène, qui déclenche une réaction d'allergie. II s'agit d'un processus immunologique mettant justement en oeuvre la voie d'activation du complément qui ne se produit que lorsqu'un allergène est présent et qui ne touche que les sujets sensibilisés. Selon un autre des aspects de l'invention, les compositions ou les activateurs de la production endogène de Protectin ou d'un précurseur de Protectin sont destinés, tout en n'éliminant pas la réaction allergique, à en limiter les conséquences délétères au niveau des tissus cutanés en empêchant les protéines du complément de venir détruire les membranes cellulaires du tissu infiltré.

Selon une variante de l'invention, les compositions et/ou les activateurs de la production de Protectin ou d'un précurseur de Protectin seront utilisés pour prévenir et/ou diminuer l'apparition de cheveux ou de poils gris ou blancs chez l'être humain, appelée canitie. En effet, on sait que lors du blanchissement des cheveux ou des poils, les mélanocytes sont détruits par le système immunitaire. L'utilisation d'un activateur de la production de Protectin ou de précurseur de Protectin aidera les mélanocytes à se protéger de cette agression.

Dans certaines situations même, il a été montré qu'une composante inflammatoire incluant des infiltrats perifolliculaires pouvait entraver la pousse des cheveux, voire être responsable de leur chute. Dans ces situations dites alopeciques, l'activateur ou la composition seront destinés au soin des cuirs chevelus caractérisés par une chute anormalement élevée de cheveux pouvant entraîner un dégarnissement. Selon une autre variante de l'invention, les compositions et/ou les activateurs de la production de Protectin ou de précurseur de Protectin seront utilisés pour prévenir et/ou diminuer la chute des cheveux, et/ou pour améliorer la densité capillaire, en diminuant l'activité de la cascade du complément, responsable en partie de la rigidification de la gaine folliculaire (Mahé et al, Int. J. Dermatol.).

Un autre des objets de l'invention est l'utilisation d'au moins un activateur de la production de Protectin ou précurseur de Protectin pour la préparation d'une composition destinée à être appliquée sur la peau, les muqueuses et/ou le cuir chevelu, ladite composition et/ou l'activateur étant destinés au traitement d'au moins une affection et/ou d'au moins un signe associé à une affection choisie parmi l'herpès, le psoriasis, l'allergie, la dermatite séborrhéique, les affections provoquées par Pityrosporum ovale ou Malassezia, notamment Malassezia furfur, les ulcères chroniques de la peau, l'urticaire et les maladies immunologiques, notamment les dérèglements du système d'activation du complément. Plus spécifiquement, l'activateur ou la composition le contenant sont destinés à prévenir ou diminuer les signes cutanés associés à ces affections, en particulier aux peaux allergiques.

Avantageusement, les compositions ou les activateurs selon l'invention seront utilisés chez les sujets herpétiques, en traitement de relais entre deux poussées éruptives, ces poussées éruptives se traduisant notamment par une réaction communément appelée "bouton de fièvre". Selon encore un autre aspect, le modulateur de la production de Protectin est utilisé dans des compositions destinées au traitement du vitiligo, notamment en traitement d'appoint et/ou pour diminuer certains signes associés au vitiligo.

L'invention a donc également pour objet des compositions cosmétiques, dermato-cosmétiques ou dermatologiques contenant au moins un activateur de la production endogène de Protectin ou d'un précurseur de Protectin.

L'activateur de la production de Protectin ou de précurseur de Protectin peut être toute substance ou tout mélange de substances susceptibles de stimuler la synthèse de cette glycoprotéine par les cellules, en particulier par les cellules cutanées. II peut s'agir notamment d'extraits d'organismes mono ou pluricellulaires, tels que des extraits végétaux complexes ou purifiés, ou de composés chimiquement définis, ainsi que les mélanges de ceux-ci.

Avantageusement, l'activateur de production de Protectin ou de précurseur de Protectin sera choisi dans le groupe comprenant: les extraits de bactéries, en particulier de bactéries filamenteuses non photosynthétiques, les bactéries lactiques et leurs extraits, en particulier des genres Lactobacillus ou Bifidibacterium, les extraits de Ginseng, en particulier les saponines telles que les ginsenosides, en particulier les ginsenosides Rb1 et Rg1, leurs dérivés et leurs mélanges.
De préférence, si l'activateur est un extrait total de *Vitreoscilla filiformis,* il est associé à au moins un autre activateur de la production de Protectin ou de précurseur de Protectin. En effet, les extraits de Vitreoscilla filiformis sont particulièrement utiles pour stimuler la production endogène de protectin par les kératinocytes. lls seront avantageusement associés à des composés capables de stimuler la production endogène de Protectin ou d'un précurseur de Protectin soit par les kératinocytes, soit par d'autres types cellulaires cutanés, en particulier par les fibroblastes. Avantageusement, la composition contient au moins un activateur de la production de Protectin ou d'un précurseur de Protectin par les kératinocytes, associé à au moins un activateur de la production de Protectin ou d'un précurseur de Protectin par les fibroblastes. En particulier, l'activateur de la production de la Protectin ou précurseur de Protectin est choisi parmi les extraits de bactéries filamenteuses non photosynthétiques et l'activateur de production de Protectin ou précurseur de Protectin par les fibroblastes est choisi dans le groupe comprenant les extraits de ginseng et les ginsenosides.

Par extrait de bactéries filamenteuses non photosynthétiques, on entend aussi bien le surnageant de culture desdites bactéries, la biomasse obtenue après culture desdites bactéries ou encore les extraits de la biomasse obtenus par traitement de cette biomasse.
Les extraits de bactéries selon l'invention sont préparés à partir de bactéries filamenteuses non photosynthétiques telles que définies selon la classification du Bergey's Manual of Systematic Bacteriology (vol. 3, sections 22 et 23, 9°édition, 1989), parmi lesquelles on peut citer les bactéries appartenant à l'ordre des Beggiatoales, et plus particulièrement les bactéries appartenant aux genres Beggiatoa, Vitreoscilla, Flexithrix ou Leucothrix.

Les bactéries qui viennent d'être définies et dont plusieurs ont déjà été décrites ont généralement un habitat aquatique et peuvent être trouvées notamment dans des eaux marines ou dans des eaux thermales. Parmi les bactéries utilisables, on peut citer par exemple :
*Vitreoscilla filiformis* (ATCC 15551)
*Vitreoscilla beggiatoïdes* (ATCC 43181)
*Beggiatoa alba* (ATCC 33555)
*Flexithrix dorotheae* (ATCC 23163)
*Leucothrix mucor* (ATCC 25107)
*Sphaerotilus natans* (ATCC 13338)

Pour préparer l'extrait selon l'invention, on peut cultiver lesdites bactéries selon les méthodes connues de l'homme du métier, puis les séparer de la biomasse obtenue, par exemple par filtration, centrifugation, coagulation et/ou lyophilisation.
On peut notamment préparer les extraits utilisables selon l'invention, selon le procédé décrit par la demanderesse dans la demande de brevet WO-A-93/00741.
Ainsi, après culture, les bactéries sont concentrées par centrifugation. La biomasse obtenue est autoclavée. Cette biomasse peut être lyophilisée pour constituer ce que l'on appelle l'extrait lyophilisé. Toute méthode de lyophilisation connue de l'homme du métier est utilisable pour préparer cet extrait.

La fraction surnageante de cette biomasse peut également être filtrée dans un récipient stérile pour éliminer les particules en suspension. On obtient ainsi l'extrait dénommé par ailleurs dans le texte extrait aqueux.
La demande EP 604631 décrit des extraits de bactéries filamenteuses comme agent stimulant l'immunité non spécifique. Or de manière inattendue, la demanderesse a maintenant montré que des extraits de telles bactéries peuvent être utilisés comme agents stimulant la synthèse endogène de Protectin ou précurseur de Protectin. Selon d'autres modes de mise en oeuvre de l'invention, on peut utiliser des extraits décrits dans EP765567.

Par Ginseng, on entend ici la plante Panax ginseng, ainsi que les plantes de la même famille telles que le Panax Notoginseng (San Qi), Panax quinquefolium ou Panax japonicum; il est utilisé dans la médecine traditionnelle pour ses propriétés toniques et fortifiantes, en particulier en gérontologie. De nombreux effets ont été allégués, et notamment une stimulation de l'immunité cellulaire et humorale après injection. On utilise généralement des extraits de racine de cette plante, dont il existe deux types principaux, le ginseng blanc et le ginseng rouge. Les racines contiennent 0,5 à 3% de saponines appelées ginsenosides (ou panaxosides), un mélange complexe de glycosides de dammarane, dont beaucoup présentent un squelette protopanaxadiol ou protopanaxatriol. Les principaux ginsenosides sont notés Rb1, Rb2, Rg1. Les extraits de ginseng contiennent d'autres composés tels que des sesquiterpènes ou des alcynes-alcools. Des extraits de ginseng particulièrement adaptés à la mise en oeuvre de l'invention sont enrichis en ginsenosides Rb1 et Rg1, tels que les extraits de Panax notoginseng. Avantageusement, les compositions selon l'invention contiennent au moins un ginsenoside Rb1 (répondant à la formule brute C₅₄H₉₂O₂₃) ou un ginsenoside Rg1 (de formule brute C₄₂H₇₂O₁₄).

Les substances ou composés mentionnés ont en effet en commun une capacité à stimuler la synthèse endogène de Protectin ou précurseur de Protectin, comme cela a été trouvé de façon surprenante par la demanderesse dans le cadre de la présente invention.
Cette propriété a ainsi été mise en évidence dans un premier temps par la technique dite des "c-DNA array", qui permet d'identifier les cibles transcriptomiques des composés et d'évaluer l'induction d'expression des ARN messagers correspondant à la protectin.

L'identification de l'induction de la synthèse d'ARNm par les composés testés a été complétée par des études au moyen d'anticorps spécifiques de la protectin, qui ont confirmé l'augmentation de la production de la protéine, en particulier par des kératinocytes ou des fibroblastes cutanés.
On peut ainsi déterminer des agents actifs sur ces différents types de cellules cutanées, ces agents étant avantageusement utilisés en association pour améliorer leur efficacité.

La stimulation de la synthèse endogène de Protectin ou de précurseur de Protectin est particulièrement avantageuse, puisqu'on aura ainsi la présence de la protéine de protection in situ, sans avoir à appliquer une protéine exogène, qui risquerait d'être dégradée par les enzymes cutanés, voire rejetée par l'organisme par une réaction de rejet immunitaire.

L'homme du métier est ainsi capable, de déterminer d'autres activateurs de la production de Protectin ou de précurseur de Protectin adaptés à la mise en oeuvre de l'invention.

L'invention a également pour objet un procédé de sélection d'un produit capable de protéger les membranes cellulaires, caractérisé en ce qu'il comporte une étape consistant à mettre le produit à tester en contact avec des cellules cutanées et à comparer le taux de protectin ou de précurseur de protectin produit par lesdites cellules cutanées, avec le taux de production de cellules cutanées témoin n'ayant pas été en contact avec le produit à tester.

En particulier, le produit à tester sera mis en contact avec une culture de cellules cutanées. Des exemples non limitatifs de telles culture sont des cultures de fibroblastes à confluence ou des cultures de kératinocytes, ou des peaux reconstruites (par exemple Episkin®).
On compare alors la quantité d'ARN messager codant pour la protectin synthétisé par ces cultures avec celles d'une population témoin, de cellules cultivées dans les mêmes conditions à l'exception du produit à tester. Si le taux d'ARN m est supérieur d'un facteur d'au moins 1,5, notamment au moins deux fois supérieur à celui de la culture témoin, le produit à tester est considéré comme un activateur de la production de Protectin ou de précurseur de Protectin et utilisable selon l'invention. De préférence, la synthèse des ARN messagers est augmenté d'un facteur supérieur ou égal à 2. De préférence, la technique est mise en oeuvre avec un témoin interne, tel que l'actine, pour vérifier la spécificité de l'induction.

Cette première étape est avantageusement complétée par des essais montrant une augmentation au niveau de la production de la protéine.

Le produit sélectionné protège notamment les membranes cellulaires contre les effets non spécifiques de l'activation du complément. Le procédé de sélection selon l'invention sera ainsi particulièrement adapté pour sélectionner des produits, plus ou moins complexes, à effets apaisant ou anti-irritant.

Selon un mode de réalisation avantageux du procédé, il permet de sélectionner des extraits cellulaires enrichis en activité protectin comme par exemple des tapis épidermiques utilisables comme greffes de peaux mieux tolérées. On met ainsi en contact un tapis de cellules épidermiques avec au moins un activateur de protectin ou de précurseur de Protectin tel que défini précédemment pendant un temps adapté au déclenchement de la stimulation, puis ces tapis prétraités sont utilisés pour des greffes de peaux qui seront mieux tolérées que des greffons standards.

Selon encore un autre aspect l'invention concerne un procédé dans lequel on stimule des cellules en présence des différents extraits sélectionnés inducteurs de Protectin ou de précurseur de Protectin, puis les cellules stimulées sont utilisées, tout ou partie comme source de Protectin ou de précurseur de Protectin en vue d'une utilisation directe ou sous une forme purifiée. L'invention a donc également pour objet des cellules stimulées in vitro par des activateurs de Protectin ou de précurseur de Protectin et des compositions contenant de telles cellules stimulées ou des extraits purifiés à partir de cellules ainsi stimulées ou de leur milieu de culture.

Un autre des objets de l'invention est l'utilisation d'au moins un composé choisi parmi les extraits de bactéries, en particulier de bactéries filamenteuses non photosynthétiques, les extraits de Notoginseng ou de Ginseng, en particulier les saponines telles que les ginsenosides Rb1 et Rg1, leurs dérivés et leurs mélanges pour la préparation d'une composition destinée à stimuler la production de Protectin ou d'un précurseur de Protectin par les cellules cutanées.

Les compositions utilisées selon l'invention peuvent se présenter sous toutes les formes adaptées aux applications envisagées, notamment par voie orale ou topique, dans les domaines cosmétique et dermatologique.

La composition selon l'invention peut ainsi être appliquée sur toute zone cutanée du corps, les cheveux les ongles ou les muqueuses et se présenter sous toute forme galénique adaptée par l'homme du métier. Elle peut se présenter notamment sous forme d'une solution ou d'une suspension aqueuse ou huileuse, d'une émulsion huile-dans-eau ou eau-dans-huile ou multiple, d'une . émulsion siliconée, d'une microémulsion ou nanoémulsion, d'un gel aqueux ou huileux ou d'un produit anhydre liquide, pâteux ou solide. Pour une application topique sur la peau, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydre, ou encore de microcapsules ou microparticules, ou de dispersions vésiculaires de type ionique et/ou non ionique. Elle peut également être présentée sous forme de patch ou de système à libération contrôlée. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.

Pour les yeux, la composition peut se présenter sous forme de gouttes et pour l'ingestion, elle peut se présenter sous forme de capsules, de granulés, de gels, de pâtes à mâcher, de sirops ou de comprimés.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des masques à laisser poser sur la peau ou les cheveux, des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosacée, le psoriasis, les lichens, les prurits sévères.

Les compositions selon l'invention peuvent également consister en des préparations solides constituant des savons ou des pains de nettoyage.

Les compositions peuvent aussi être conditionnées sous forme de composition pour aérosol comprenant également un agent propulseur sous pression.

La composition selon l'invention peut aussi être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, etc.

La composition peut aussi être à usage bucco-dentaire, par exemple une pâte dentifrice. Dans ce cas, la composition peut contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition. De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les Bentones®, les sels métalliques d'acides gras comme les stéarates d'aluminium et la silice hydrophobe, éthylcellulose, polyéthylène.

La quantité d'activateur de Protectin ou de précurseur de Protectin présent dans les compositions selon l'invention sera adaptée par l'homme du métier pour obtenir l'activité protectrice recherchée, selon le type d'activateur utilisé. A titre indicatif, la quantité d'activateur dans les compositions sera comprise entre 0,001% et 10% en poids par rapport au poids total de la composition, de préférence de 0,01 à 5%; en particulier elle sera d'au moins 0,1 %.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention la composition peut associer au moins un activateur de la production endogène de Protectin ou de précurseur de Protectin à d'autres agents actifs destinés notamment à la prévention et/ou au traitement des affections cutanées. Parmi ces agents actifs, on peut citer à titre d'exemple:
- les agents hydratants qui peuvent être :

- soit un composé agissant sur la fonction barrière, en vue de maintenir l'hydratation du stratum corneum, ou un composé occlusif. On peut citer les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ;
- soit un composé augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine ;
- les agents modulant la différenciation et/ou la prolifération cutanée tels que l'acide rétinoïque et ses isomères, le rétinol et ses esters, la vitamine D et ses dérivés; les agents stimulant la prolifération des fibroblastes peuvent être choisis par exemple parmi les protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine® ) ; et les hormones végétales telles que les giberrellines et les cytokinines.
   Les agents stimulant la prolifération des kératinocytes, utilisables dans la composition selon l'invention, comprennent notamment les rétinoïdes tels que le rétinol et ses esters, dont le palmitate de rétinyle; les extraits de tourteaux de noix commercialisés par la société GATTEFOSSE; et les extraits de Solanum tuberosum commercialisés par la société SEDERMA.
   Les agents stimulant la différenciation des kératinocytes comprennent par exemple les minéraux tels que le calcium ; l'extrait de lupin commercialisé par la société SILAB sous la dénomination commerciale Photopréventine®; le beta-sitosteryl sulfate de sodium commercialisé par la société SEPORGA sous la dénomination commerciale Phytocohésine®; et l'extrait de maïs commercialisé par la société SOLABIA sous la dénomination commerciale Phytovityl®.
- le lycopène, les caroténoïdes et la lutéine
- les agents dépigmentants comme par exemple les composés suivants : l'acide kojique; l'acide ellagique; l'arbutine et ses dérivés tels que ceux décrits dans les demandes EP-895 779 et EP-524 109; l'hydroquinone ; les dérivés d'aminophénol tels que ceux décrits dans les demandes WO 99/10318 et WO 99/32077, et en particulier le N-cholestéryloxycarbonyl-para-aminophénol et le N-éthyloxycarbonyl-para-aminophénol; les dérivés d'iminophénol, en particulier ceux décrits dans la demande WO 99/22707; l'acide L-2-oxothiazolidine-4-carboxylique ou procystéine, ainsi que ses sels et esters; l'acide ascorbique et ses dérivés, notamment le glucoside d'ascorbyle; et les extraits de plantes, en particulier de réglisse, de mûrier et de scutellaire, sans que cette liste soit limitative. La composition selon la présente invention comprenant les agents dépigmentants cités ci-dessus est avantageusement destinée à la prévention ou au traitement des hyperpigmentations, en particulier des taches pigmentaires liées au vieillissement de la peau.
- les agents pro-pigmentants: on peut citer l'extrait de pimprenelle (Sanguisorba officinalis) commercialisé par la société MARUZEN et les extraits de chrysanthème (Chrysanthemum morifolium). La composition renfermant les agents pro-pigmentants cités précédemment est de préférence destinée au traitement de la canitie.
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anti-inflammatoires susceptibles d'inhiber les principales enzymes impliquées dans le processus inflammatoire (cascade de l'acide arachidonique), à savoir: les phospholipases A2 (PLA2) ; les lipoxygénases (Lox) ; et les prostaglandines humaines synthétases (PGHS). Parmi les matières premières efficaces pour inhiber au moins une de ces enzymes, on peut citer de façon non limitative les actifs suivants : les triterpènes pentacycliques et les extraits de plante(ex : Glycyrrhiza glabra) en contenant comme l'acide β-glycyrrhétinique et ses sels et/ou ses dérivés (l'acide glyyrrhetique monoglucuronide, le stearyl glycyrrhetinate, l'acide 3- stéaroyloxy glycyrrhetique), l'acide ursolique et ses sels, l'acide oléanolique et ses sels, l'acide bétulinique et ses sels, l'extrait de Paeonia suffruticosa et / ou lactiflora, l'huile de calophilum, les sels de l'acide salicylique et en particulier le salicylate de zinc, les phycosaccharides anti-inflammatoires (hydrolysed algin ou hydrolysed algin and zinc sulfate) de la société Codif, la phlorogine (laminaria saccharina) de Secma, l'huile de Canola, de Tamanu, de calophillum, l'β-bisabolol et les extraits de camomille, l'allantoine, le Sépivital EPC (diesterphosphorique de vitamine E et C) de Seppic, les huiles insaturées en oméga 3 telles que les huiles de rosier musca, de cassis, d'ecchium, de poisson, l'omega plancton (plancton extract) de Secma, le lipacide C8G ( capriloyl glycine) de Seppic, le Seppicalm VG (sodium palmitoylproline and nymphea alba) de Seppic, l'extrait d'epilobe, l'extrait du pygeum, le Soothex ( extrait de boswellia serrata) de Quest, le phytoplenolin (extrait de centipeda cunnighami) de Bio-Botanica, l' hélioxine (extrait d'hélianthus annuus) de Silab, la Sensiline (linum usitatissimum) de Silab, les tocotrienols, les extraits de Cola nitida, le piperonal, l'extrait de clou de girofle, l'extrait d'épilobe (Epilobium Angustifolium) l'aloe vera, la bacocalmine (extrait de bacopa moniera) de SEDERMA, les phytostérols, la cortisone, l'hydrocortisone, l'indometacine la béta methasone et le NDGA.
- les agents anesthésiques tels que le chlorhydrate de lidocaïne et ses dérivés ;
- les agents antiprurigineux comme la thénaldine, la triméprazine ou la cyproheptadine;
- les agents kératolytiques tels que les acides alpha- et bêta-hydroxycarboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que la vitamine E ou l'alpha-tocophérol ses dérivés ou ses esters tels que l'acétate de tocophéryle, certains chélatants de métaux ou l'acide ascorbique et ses esters; les bioflavonoïdes; le co-enzyme Q10 ou ubiquinone; certaines enzymes comme la catalase, les superoxyde dismutase, la lactoperoxydase, le glutathion peroxydase et les quinones réductases; le glutathion; le benzylidène camphre; les benzylcyclanones; les naphtalénones substituées; les pidolates; le phytantriol; le gamma-oryzanol; les lignanes; et la mélatonine.
- les anti-séborrhéiques tels que la progestérone;
- les actifs anti-chute tels que l'aminexil décrit dans la demande EP 540629, ou des agonistes des canaux potassium incluant le minoxidil ou 2,4-diamino 6-piperidinopyrimidine et ses dérivés, les inhibiteurs de lipoxygénase tels que décrits dans EP 648488, les inhibiteurs de bradyknine décrits notamment dans EP 845700, les prostaglandines et leurs dérivés, les vasodilatateurs et les anti-androgènes,
- les inhibiteurs de 5α-réductase choisis notamment parmi les rétinoïdes, et en particulier le rétinol ;le soufre et les dérivés soufrés ;les sels de zinc tels que le lactate, le gluconate, le pidolate, le carboxylate, le salicylate et/ou le cystéate de zinc ;le chlorure de sélénium ;la vitamine B6 ou pyridoxine ;le mélange de capryloyl glycine, de sarcosine et d'extrait de cinnamomum zeylanicum commercialisé notamment par la société SEPPIC sous la dénomination commerciale Sepicontrol A5® ;un extrait de Laminaria saccharina commercialisé notamment par la société SECMA sous la dénomination commerciale Phlorogine® ;un extrait de Spiraea ulmaria commercialisé notamment par la société SILAB sous la dénomination commerciale Sebonormine® ;des extraits de végétaux des espèces Arnica montana, Cinchona succirubra, Eugenia caryophyllata, Humulus lupulus, Hypericum perforatum, Mentha piperita, Rosmarinus officinalis, Salvia officinalis et Thymus vulgaris, tous commercialisés par exemple par la société MARUZEN ; un extrait de Serenoa repens commercialisé notamment par la société EUROMED ; des extraits de plantes du genre Silybum ; des extraits végétaux contenant des sapogénines et en particulier les extraits de Dioscorées riches en diosgénine ou hécogénine ; et des extraits d'Eugenia caryophyllata contenant de l'eugénol et du glucoside d'eugényle. Ces agents seront présents en particulier dans des compositions destinées à prévenir et/ou traiter la séborrhée et/ou la chute des cheveux.
- les antipelliculaires comme l'octopirox ou la pyrithione de zinc ;
- les antiacnéiques comme l'acide rétinoïque ou le peroxyde de benzoyle ;
- les extraits végétaux ou d'origine microbienne,

Avantageusement, la composition selon l'invention comprendra des nanosphères ou des microsphères, telles que décrits par exemple dans les demandes de brevet EP447318, EP557489, EP1151741, EP1201219 ou WO 97/12602

Selon un mode de réalisation particulier de l'invention, les compositions contiennent au moins un activateur de la production de Protectin ou d'un précurseur de Protectin en association avec au moins un composé, notamment un principe actif, connu comme ayant un effet secondaire irritant.

Les produits irritants auxquels s'applique l'invention sont notamment les parfums, les tensioactifs (ioniques ou non-ioniques), les conservateurs, certains filtres solaires, les solvants organiques, les solutions alcooliques et certains actifs cosmétiques, pharmaceutiques ou dermatologiques.

En particulier, les produits à effet secondaire irritant sont choisis parmi les α-hydroxy-acides (glycolique, lactique, malique, citrique, tartrique, mandélique ), les β-hydroxy-acides (acide salicylique et ses dérivés), les α-céto-acides, les β-céto-acides, les rétindides (rétinol et ses esters, rétinal, acide rétinoïque et ses dérivés, rétinoïdes, notamment ceux décrits dans les documents FR-A-2 570 377, EP-A-199636, EP-A-325540, EP-A-402072), les anthralines (dioxyanthranol), les anthranoïdes, les peroxydes, le minoxidil, les sels de lithium, les antimétabolites, la vitamine D et ses dérivés, les teintures ou colorants capillaires (paraphénylènediamine et ses dérivés, les aminophénols), les solutions alcooliques parfumantes (parfums, eaux de toilette, après rasage, déodorants), les agents antitranspirants (certains sels d'aluminium), les actifs dépilatoires ou de permanentes (thiols), les dépigmentants (hydroquinone), les actifs antipoux (pyréthrine).

L'invention a également pour objet un procédé cosmétique pour améliorer la résistance de la peau aux effets de l'activation non spécifique du complément, caractérisé en ce qu'on applique topiquement au moins un activateur de la production de Protectin ou d'un précurseur de Protectin, susceptible d'être obtenu par le procédé de sélection décrit plus haut.

### Exemple 1 - Mesure de l'augmentation de la synthèse de protectin (CD 59) par Vitreoscilla filiformis

### I. Matériel et méthodes.

### - Cultures cellulaires et traitement :

Les études sont réalisées sur kératinocytes épidermiques humains normaux en culture. Les kératinocytes sont cultivés en milieu SFM sans extrait pituitaire et EGF.
Les extraits de Vitreoscilla filliformis ATCC1551 obtenus suivant les protocoles décrits dans le brevet WO-A-93/00741 ont été appliqués sur les cellules à la concentration de 0.1 % (p/v). Le temps de contact a été de 18 heures.

### -cDNA array:

La méthodologie utilisée est celle préconisée par Clontech (Palo Alto, USA). L'extraction/purification de l'ARN total de chaque culture a conduit à l'isolement de quantités d'ARN total de l'ordre de 100 à 150 µg. Les solutions d'ARN total sont traitées par la DNAse I pour éliminer toute trace d'ADN contaminant, selon les recommandations du fournisseur. La qualité de l'ARN a ensuite été vérifiée sur gel d'agarose et les solutions d'ARN ont été ajustées à 1 µg/ml.
L'étape suivante a été la purification des ARN messagers (ARNm) par hybridation des extrémités poly(A) des ARNm à des amorces oligo(dT) biotinylées et capture sélective sur billes de streptavidine, selon le protocole Atlaspure (Clontech). Les sondes ADN marquées au ³³P ont été réalisées par reverse-transcription des ARNm liés sur billes de poly(dT), à l'aide d'un pool de primers spécifiques des séquences immobilisées sur les membranes ( arrays ), en présence de (α³³P)-dATP. Cette étape a utilisé les réactifs et le protocole préconisé par Clontech. Les sondes marquées ont été purifiées par chromatographie sur colonne d'exclusion et la qualité et l'équivalence des sondes marquées a été évaluée par comptage en scintillation liquide.
Des membranes de cDNA array ont été pré-traitées puis les cDNAs immobilisés sur chaque membrane ont été hybridés (68°C, une nuit) aux sondes marquées correspondantes. Les filtres ont ensuite été lavés et placés dans des sacs plastiques individuels pour analyse. L'analyse a eu lieu par quantification directe de la radioactivité des spots à l'aide d'un phospholmager Cyclone (Packard). Les résultats sont exprimés en unités relatives d'expression (RE, radioactivité du spot correspondant à chaque gène, corrigé du bruit de fond et des différences d'intensité de marquage des sondes).

### - RT-Q-PCR:

Les couples de primer utilisés dans cette étude sont la CD 59 glycoprotein precursor (CD59, taille du fragment amplifié 321 pb) et des amorces de séquence d'actine (450 pb) Les ARN totaux sont extraits à l'aide de Tri-Reagent selon le protocole préconisé par le fournisseur. Elle est suivie d'une nouvelle extraction par du chloroforme et d'une précipitation à l'isopropanol. Les traces d'ADN potentiellement contaminantes sont éliminées par traitement avec le système DNA-free (Ambion). La réaction de réverse-transcriptase est ensuite réalisée en présence de l'amorce oligo(dT) et de l'enzyme Superscript II (Gibco). Cette étape est suivie d'une quantification, par fluorescence, de l'ADNc synthétisé et ajustement des concentrations à 150 ng/ml. Une nouvelle quantification de chaque ADNc, après dilution finale, est réalisée avant la réaction de PCR.

Les réactions de PCR (polymérase chain réactions) ont été réalisées par PCR quantitative avec le système « Light Cycler » (Roche Molecular Systems Inc.) et selon les procédures recommandées par le fournisseur. Le mélange réactionnel (10 µl final) introduit dans les capillaires d'un thermocycleur est composé de 2.5 µl d'ADNc, des amorces des deux marqueurs, du mélange réactionnel (Roche) contenant l'enzyme taq DNA polymérase, le marqueur SYBR Grenn l. Les conditions de la PCR sont les suivante : activation de 10 min. à 95°C, réactions de PCR en 40 cycles, fusion 5 sec à 95 °C puis 5 sec. à 60 °C.
L'analyse de fluorescence dans l'ADN amplifié est mesurée en continu au cours des cycles de PCR. La valeur moyenne de l'expression relative (RE) est exprimée en Unités Arbitraires (UA) calculées à partir des valeurs de cycles de deux PCRs indépendantes selon la formule suivante : (1/2 ^{nombre de cycles}) × 10⁶.
Les résultats d'expression de CD 59 sont comparées à celle de l'actine afin de prendre en compte les éventuels écarts de concentration cellulaire dans les deux populations cellulaires.

### - Etude en cytométrie de flux:

Les cellules sont pré-cultivées à forte densité pendant 48 heures puis traitées ou non par le produit à l'essai pendant 24 ou 48 heures. Les cellules sont trypsinisées puis rincées par une solution PBS/2 % SVF. Les cellules sont transférées en tubes Eppendorf et centrifugées 5' à 1500 rpm. Les cellules sont fixées par une solution PBS-formol à 4 % final, 30' à température ambiante et à l'obscurité. elles sont ensuite perméabilisées à l'aide d'une solution 0.1 % Triton-X100 / 0.1 % citrate. Le marquage des cellules est réalisé en présence d'anticorps anti-CD 59 (TEBU SOD-110) et anti-human-CD59-FITC (Biosciences 555763) selon les indications du fournisseur. L'analyse des échantillons est réalisée par cytométrie (cytomètre FACSCAN, logiciel Cell Quest ; Becton-Dickinson) sur la population totale. Les statistiques sont réalisées sur 10 000 cellules de chaque échantillon. Les résultats sont exprimés en intensité de fluorescence (IF) correspondant à la quantité relative de chaque marqueur par cellule dans une population totale de 10 000 cellules analysées.

### II. Résultats:

### 2.1 ) Etude de cDNA macroarray

Résultats sur culture de kératinocytes humains (intensité de signal)

| Produit testé | Cellules Témoin | Cellules exposées à l'extrait | Comparaison entre cellules témoin et cellules exposées à (en %) |
|---|---|---|---|
| Extrait de V. filiformis à 0,1% | 13.1 | 39.3 | 495 (x 4.95) |

On constate que la population cellulaire constituée de kératinocytes exposée à l'extrait de bactérie filamenteuse non photosynthétique non fructifiante produit significativement plus d'ARNm de CD59 que la population témoin (environ 5 fois plus).

### 2.2) Etude de Q RT-PCR

Les résultats ci-dessous sont ceux obtenus sur culture de kératinocytes humains.

### Résultats sur la CD59

| Traitement | Cycles Actine | Cycles CD59 | RE*Actine (UA) | RE* CD59 (UA) | CD59/ Actine | % Témoin |
|---|---|---|---|---|---|---|
| Témoin | 16.30 | 19.82 | 12.31 | 1.12 | 0.091 | 100 |
| | 16.32 | 19.71 | | | | |
| ViFi | 17.45 | 19.86 | 5.55 | 1.01 | 0:181 | 199 |
| | 17.47 | 19.99 | | | | |
| (ViFi : *Vitreoscilla filliformis)* | | | | | | |
| RE : expression relative exprimée en unités arbitraires. | | | | | | |

Ces résultats confirment que l'extrait ViFi induit d'un facteur 2 la production de la CD59

### 2.3) Etude de cytométrie de flux

Effet du traitement sur la quantité relative de CD59: les résultats ci-dessous sont ceux obtenus sur culture de kératinocytes humains.

Quantité relative de CD 59 dans des kératinocytes témoins et dans des kératinocytes traités par l'extrait (0.1 %, p/v) pendant 48 heures.

| Traitement | Intensité Fluo | % |
|---|---|---|
| Témoin | 114.4 | 100 |
| Extrait ViFi | 207.7 | 182 |
| (ViFi: *Vitreoscilla filliformis*) | | |

Dans les conditions expérimentales, l'extrait testé a stimulé nettement l'expression de la protéine CD59 (x1.8).

### Exemple 2: augmentation de la synthèse de CD59 par des extraits de Ginseng

L'étude est réalisée sur des fibroblastes normaux humains. Les fibroblastes dermiques sont cultivés en milieu DMEM (Life Technology, ref. 21969035) contenant de la L-glutamine (2 mM, Life Technology, ref. 25030024), de la pénicilline à 50 Ul/ml et streptomycine à 50 µg/ml (Life Technology, ref. 15070063) et du sérum de veau foetal à 1% (v/v, Life Technology, ref. 10106151). Une fraction d'un extrait de racines de Panax notoginseng (San Qi) ne comprenant que les saponines est testée pour son activité selon le protocole décrit à l'exemple précédent par la technique de cDNA array sur fibroblastes. On teste également les deux saponines principales de l'extrait.

Les produits sont les suivants:
1- une fraction d'un extrait de Panax notoginseng (San Qi) comprenant 6 ginsenosides dans les proportions suivantes:
   - Ginsenoside Rb1 : 43%
   - Ginsenoside Rb2 : 1 %
   - Ginsenoside Rc : traces
   - Ginsenoside Rd : 13 %
   - Ginsenoside Re : 9 %
   - Ginsenoside Rg1 : 33 %
2- le Ginsenoside Rb1 (fournisseur Extrasynthèse)
3- le Ginsenoside Rg1 (fournisseur Extrasynthèse)

Tous les produits sont testés à une concentration finale de 15 microgrammes/ml

Les résultats sont rapportés dans le tableau suivant:

| Produit testé | Fibroblastes témoin (RE) | Fibroblastes exposés à l'extrait (RE) | Comparaison de la population témoin et de la population exposée à l'extrait (en %) |
|---|---|---|---|
| San Qi | 6.1 | 13.0 | 214 |
| Rb1 | 6.1 | 13.8 | 227 |
| Rg1 | 6.1 | 16.8 | 277 |

L'extrait total de ginseng et les ginsenosides Rb1 et Rg1 modifient de façon significative l'expression des ARN messagers codant pour la protéine CD59.

Idéalement, si l'on veut stimuler à la fois les kératinocytes et les fibroblastes cutanés à produire de la Protectin ou un précurseur de Protectin, on combinera le San Qi ou ses ginsenosides (actifs sur fibroblastes ) avec l'extrait de Vitreoscilla filiformis (plutôt actif sur les kératinocytes).

### Exemple 3: Formulation pour application sur le cuir chevelu

| **Lotion non rincée** | |
|---|---|
| Extrait de Vitreoscilla filiformis | 0,2 g |
| Aminexil | 1,5 g |
| Propylène glycol | 22,8 g |
| Ethanol 95° | 55,1 g |
| Eau purifiée | qsp 100 g |

| **Lotion non rincée** | |
|---|---|
| Extrait de Vitreoscilla filiformis | 0,1g |
| Extrait de San Qi | 0,1 g |
| Propylène glycol | 22,8 g |
| Ethanol 95° | 55,1 g |
| Eau purifiée | qsp 100 g |

| **Lotion anti-chute** | |
|---|---|
| Extrait de Vitreoscilla filiformis | 0,1 g |
| Minoxidil | 2 g |
| Propylène glycol | 22,8 g |
| Ethanol 95° | 55,1 g |
| Eau purifiée | qsp 100 g |

### Exemple 4: Crème régénératrice

- Extrait de Vitreoscilla filiformis de l'exemple 1 (extrait lyophilisé) 0,1 %
- extrait de San Qi 0,05 %
- Carbomer 940® (acide polyacrylique réticulé) 0,3 %
- Triéthanolamine 0,3 %
- Acide stéarique 3,0 %
- Alcool cétylique 2,0 %
- Monostéarate de glycérol autoémulsionnable 3,0 %
- Huile de soja 10,0 %
- Alcool de lanoline 2,0 %
- Myristate d'isopropyle 4,0 %
- 2-éthylhexanoate de cétyle et de stéaryle 4,0 %
- Perhydrosqualène 3,0 %
- Paraffine 2,0 %
- Glycérine 3,0 %
- Conservateurs 0,3 %
- Eau qsp 100

Pour préparer cette crème, on chauffe la phase aqueuse contenant la glycérine, les conservateurs et l'eau à 80°C ; on y disperse le Carbomer 940 qui est ensuite neutralisé par la triéthanolamine. La phase grasse, chauffée et homogénéisée à 80°C, est introduite sous vive agitation dans la phase aqueuse. L'extrait de l'exemple est dispersé dans 10 g d'eau et introduit à 40°C dans la crème, sous agitation. L'ensemble est refroidi jusqu'à température ambiante.
Cette crème est appliquée sur la peau du visage et du cou une à deux fois par jour.

### Exemple 5 - Crème de soin de l'érythème solaire (émulsion huile-dans-eau)

| | |
|---|---|
| Extrait de l'exemple 1 (extrait lyophilisée ) | 0,75 % |
| Stéarate de glycérol | 2,00 % |
| Polysorbate 60 (Tween 60 vendu par la société ICI) | 1,00 % |
| Acide stéarique | 1,40 % |
| Acide glycyrrhétinique | 2,00 % |
| Triéthanolamine | 0,70 % |
| Carbomer | 0,40 % |
| Fraction liquide du beurre de karité | 12,00 % |
| Huile de tournesol | 10,00% |
| Antioxydant | 0,05 % |
| Parfum | 0,50 % |
| Conservateur | 0,30 % |
| Eau | qsp 100 % |

## Revendications

1. Utilisation d'au moins un activateur de la production de Protectin ou d'un précurseur de Protectin (CD59) dans une composition cosmétique ou pour la préparation d'une composition destinée à limiter les effets secondaires de l'activation du complément sur la peau, les muqueuses et/ou le cuir chevelu.

2. Utilisation selon la revendication 1 **caractérisée en ce que** la composition est destinée à être appliquée sur la peau, les muqueuses et/ou le cuir chevelu..

3. Utilisation selon l'une des revendications 1 ou 2 **caractérisée en ce que** l'activateur ou la composition sont destinés à renforcer la résistance de la peau et/ou des follicules pileux.

4. Utilisation selon l'une des revendications 1 à 3 **caractérisée en ce que** la composition est une composition apaisante et/ou anti-irritante.

5. Utilisation selon l'une des revendications 1 à 4, **caractérisée en ce que** l'activateur ou la composition sont destinés à prévenir, diminuer ou traiter l'érythème, en particulier l'érythème solaire.

6. Utilisation selon l'une des revendications 1 à 4, **caractérisée en ce que** l'activateur ou la composition sont destinés à traiter les peaux acnéiques et séborrhéiques.

7. Utilisation selon l'une des revendications 1 à 4, **caractérisée en ce que** l' activateur ou la composition sont destinés au traitement des peaux sensibles.

8. Utilisation selon la revendication 1 **caractérisée en ce que** l'activateur ou la composition sont destinés à prévenir ou diminuer la canitie.

9. Utilisation selon la revendication 1 **caractérisé en ce que** la composition est destinée au traitement d'au moins une affection choisie parmi l'herpès, le psoriasis, l'allergie, la dermatite séborrhéique, les affections provoquées par Pityrosporum ovale, l'urticaire et les dérèglements du système d'activation du complément.

10. Utilisation d'au moins un composé choisi parmi les extraits de bactéries, en particulier de bactéries filamenteuses non photosynthétiques, les extraits de ginseng, en particulier les saponines et les ginsenosides, leurs dérivés et leurs mélanges pour la préparation d'une composition destinée à stimuler la production de Protectin ou d'un précurseur de Protectin par les cellules cutanées.

11. Composition cosmétique, dermato-cosmétique ou dermatologique **caractérisé en ce qu'**elle contient au moins un activateur de la production de Protectin ou d'un précurseur de Protectin, à la condition que si l'activateur est un extrait total de Vitreoscilla filiformis, il est associé à au moins un autre activateur de la production de Protectin ou précurseur de Protectin.

12. Composition selon la revendication 11, **caractérisée en ce que** l'activateur de la production de Protectin ou d'un précurseur de Protectin est choisi dans le groupe comprenant: les extraits de bactéries, en particulier de bactéries filamenteuses non photosynthétiques, les extraits de ginseng, en particulier les saponines et les ginsenosides, leurs dérivés et leurs mélanges.

13. Composition selon l'une des revendications 11 ou 12, **caractérisée en ce qu'**elle contient au moins un activateur de la production de Protectin ou d'un précurseur de Protectin par les kératinocytes, associé à au moins un activateur de la production de Protectin ou d'un précurseur de Protectin par les fibroblastes.

14. Composition selon la revendication 13 **caractérisé en ce que** l'activateur de la production de la Protectin ou de précurseur de Protectin par les kératinocytes est choisi parmi les extraits de bactéries filamenteuses non photosynthétiques et l'activateur de production de Protectin ou de précurseur de Protectin par les fibroblastes est choisi dans le groupe comprenant les extraits de Panax ginseng, Panax notoginseng, Panax quinquefolium et Panax japonicus.

15. Composition selon l'une des revendications 11 à 14 **caractérisée en ce qu'**elle est sous forme d'une solution ou lotion aqueuse ou hydro alcoolique, d'une suspension ou dispersion aqueuse, hydro alcoolique ou huileuse, d'une émulsion huile/eau, d'une émulsion eau/huile, d'un gel , de consistance liquide, pâteuse, semi solide ou solide

16. Composition selon l'une des revendications 11 à 15, **caractérisé en ce qu'**elle comprend des microsphères et/ou des nanosphères.

17. Composition selon l'une des revendications 11 à 16, **caractérisée en ce qu'**elle comprend au moins un actif à effet irritant.

18. Composition selon la revendication 17 **caractérisée en ce que** l'actif à effet irritant est choisi dans le groupe comprenant : les α-hydroxy-acides en particulier glycolique, lactique, malique, citrique, tartrique, mandélique, les β-hydroxy-acides (acide salicylique et ses dérivés), les α-céto-acides, les β-céto-acides, les rétindides tels que rétinol et ses esters, rétinal, acide rétinoïque et ses dérivés, les anthralines tel que le dioxyanthranol, les anthranoïdes, les peroxydes, le minoxidil, les sels de lithium, les antimétabolites, la vitamine D et ses dérivés, les teintures ou colorants capillaires tels que paraphénylènediamine et ses dérivés, les aminophénols, les solutions alcooliques parfumantes, les agents antitranspirants, certains sels d'aluminium, les actifs dépilatoires ou de permanentes, les dépigmentants, les actifs anti-poux.

19. Procédé de sélection d'un produit capable de protéger les membranes cellulaires, susceptible d'être utilisé selon l'une des revendications 1 à 10, **caractérisé en ce qu'**il comporte une étape consistant à mettre le produit à tester en contact avec des cellules cutanées et à comparer le taux de Protectin, ou de précurseur de la Protectin, produit par lesdites cellules cutanées avec le taux de production de cellules cutanées témoin n'ayant pas été en contact avec le produit à tester.

20. Procédé selon la revendication 19, **caractérisé en ce qu'**il permet de sélectionner des produits à effets anti-irritants.

21. Procédé selon la revendication 19, **caractérisé en ce qu'**il permet de sélectionner des extraits cellulaires enrichis en activité protectin comme par exemple des tapis épidermiques utilisables comme greffes de peaux mieux tolérées.

22. Procédé cosmétique pour améliorer la résistance de la peau aux effets de l'activation non spécifique du complément, **caractérisé en ce qu'**on applique topiquement au moins un activateur de la production de Protectin ou de précurseur de Protectin, susceptible d'être obtenu par le procédé selon l'une des revendications 19 ou 20.
